# EUROPÄISCHE PATENTSCHRIFT

(11) **EP 3 622 531 B1**
(45) Veröffentlichungstag und Bekanntmachung des Hinweises auf die Patenterteilung: **07.08.2024**
(21) Anmeldenummer: 18743671.2
(22) Anmeldetag: 09.05.2018
(51) Int. Cl.: G16H 80/00, G16H 10/60

(54) **DIE SICHTUNGSVORRICHTUNG ZUR SICHTUNG VON SCHADENSEREIGNISSEN**
THE SIGHTING DEVICE FOR THE TRIAGE OF DAMAGE EVENTS
LE DISPOSITIF DE VISÉE POUR LA TRIAGE D'ÉVÉNEMENTS DOMMAGEABLES

(30) Priorität: 09.05.2017 DE 102017110012
(43) Veröffentlichungstag der Anmeldung: 18.03.2020
(73) Patentinhaber: ITK Engineering GmbH, 76761 Rülzheim (DE); 3I Ingenieure Für Innovationsberatung Und Informationsmanagement, 50679 Köln (DE)
(72) Erfinder: HEUER, Stephan, 76227 Karlsruhe (DE); DE KLERK, Richard, 76187 Karlsruhe (DE); WEBER, Benedikt, 50676 Pulheim (DE); KÄSER, Benjamin, 51069 Köln (DE); SCHÜTTE, Frederik, 50679 Köln (DE)
(74) Vertreter: Dr. Langfinger & Partner
(86) Internationale Anmeldenummer: PCT/DE2018/100450
(87) Internationale Veröffentlichungsnummer: WO 2018/206057

(56) Entgegenhaltungen:
- JP-A- 2015 055 995
- JP-A- 2015 185 148
- US-A1- 2011 130 636
- US-B2- 7 629 881
- TAMMARA MASSEY ET AL: "The design of a decentralized electronic triage system", AMIA ... ANNUAL SYMPOSIUM PROCEEDINGS. AMIA SYMPOSIUM, 1 January 2006 (2006-01-01), United States, pages 544, XP055499461, Retrieved from the Internet <URL:https://www.ncbi.nlm.nih.gov/pmc/articles/PMC1839501/pdf/AMIA2006_0544.pdf> [retrieved on 20180813]
- LESLIE A LENERT ET AL: "An Intelligent 802.11 Triage Tag for medical response to disasters", AMIA ... ANNUAL SYMPOSIUM PROCEEDINGS. AMIA SYMPOSIUM, 1 January 2005 (2005-01-01), United States, pages 440, XP055499589, Retrieved from the Internet <URL:https://www.ncbi.nlm.nih.gov/pmc/articles/PMC1560742/pdf/amia2005_0440.pdf> [retrieved on 20180813]

## Beschreibung

Die vorliegende Erfindung betrifft eine Sichtungsvorrichtung zur Sichtung von Schadensereignissen.

Ein Massenanfall von Verletzten (MANV) bezeichnet eine Situation, bei der eine große Anzahl von betroffenen Personen versorgt werden muss. Je nach Ausmaß und Umfang des Massenanfalls variiert der Bedarf an Einsatzmitteln beträchtlich.

Dabei ist das erste Ziel der Bewältigung eines Massenanfalls von Verletzten eine möglichst rasche Wiederherstellung einer adäquaten Versorgungslage. Hierfür werden Einsatzmittel nicht nur aus der von dem Massenanfall von Verletzten betroffenen Region, sondern auch von Nachbarregionen alarmiert, und eine sogenannte erweiterte Führungsstruktur geschaffen, die die Einsatzmittel koordiniert.

Dabei sind wichtige Führungsfragen zu beantworten, beispielsweise wie die genaue Lagesituation ist, wie die (verletzten) Personen verteilt sind, wie viele Personen bereits gesichtet wurden, wie viele Personen noch nicht gesichtet sind, wie viel Einsatzkräfte benötigt werden, usw.

Diese Führungsfragen sind in einer Situation erhöhter Unsicherheit zu beantworten, da meist ein Mangeln an verfügbaren Ressourcen vorliegt und verlässliche Informationen über die Lage fehlen.

Hierbei kommt eine sogenannte Sichtung oder Triage zum Einsatz, ein nicht gesetzlich kodifiziertes Verfahren zur Priorisierung medizinischer Hilfeleistungen. Ohne einer strukturierten Triage besteht die Gefahr einer unethischen Selektion der betroffenen Personen. Zunächst kann eine Pre-Triage, auch Vorsichtung oder Bergungssichtung genannt, erfolgen, die eine schnelle Übersicht über die zu versorgenden Personen bieten soll.

Die Einschätzung der Erstsichter in der Pre-Triage entscheidet, welche Person zuerst versorgt werden soll. Als Richtzeit gilt dabei eine Zeit von 20 bis 60 Sekunden pro Person. Eine aufwändige Dokumentation soll dabei nicht erfolgen, vielmehr erfolgt eine Farbcodierung der Personen und es wird eine Strichliste der Personen und eine grobe Lageskizze erstellt.

Die Personen werden dabei anhand verschiedener möglicher Sichtungsalgorithmen in Sichtungskategorien eingeteilt. Weit verbreitet ist bspw. das sogenannte "Simple Triage and Rapid Treatment Schema" (STaRT-Schema), bei dem vier Kategorien zur Auswahl stehen: 1. T3/MINOR, Personen die selber laufen können; 2. T1/IMMEDIATE, Personen im lebensbedrohlichen Zustand; 3. T2/DELAYED, alle anderen Personen; und T4/DECEASED, Personen die verstorben sind.

Alternativ zum STaRT-Schema sind zudem das modifizierte mSTaRT, das BASIC-Schema und das Reverse-Triage-Verfahren bekannt. Im Ausland sind ggf. weitere Verfahren bekannt. Alternative Verfahren werden ggf. in der notärztlichen Diskussion entwickelt bzw. bestehende Verfahren weiterentwickelt.

Nachteilig an den aus dem Stand der Technik bekannten Verfahren ist, dass der Zustand der Personen mittels Verletztenanhängerkarten, auch als Triagekarten bezeichnet, dokumentiert wird und der Zustand der Personen per Funk oder direkte Meldung an eine Einsatzleitstelle übermittelt wird.

Dieser Vorgang ist zeitaufwändig und ungenau. Zudem sind die Verletztenanhängerkarten, auf denen der Zustand der Person üblicherweise mit einer Farbcodierung festgehalten wird, wobei jede Farbe für eine der Sichtungskategorien steht, in der Dunkelheit bzw. bei Regen nur schwer erkennbar. Auch können Personen von den den Erstsichtern folgenden Einsatzkräften übersehen werden. Zudem ist die notwendige Dokumentation für die Sichtenden zeitaufwändig und hält diese von der weiteren Suche nach Personen ab.

Es wäre daher wünschenswert, eine Vorrichtung zu liefern, die eine schnellere und einfachere Bergungssichtung ermöglichen würde.

Aus DE 10 2005 004 773 A1 ist ein Verfahren bekannt, dass die Bergungssichtung vereinfachen soll. Hierbei wird eine Vorrichtung bereitgestellt, die an der Person angeordnet wird und die es ermöglicht, den Zustand der Person einzugeben, wobei der eingegebene Zustand sowie ein mittels GPS erfasster Ort der Person über WLAN an einen zentralen Server übermittelt wird.

Tammara Massey et al., AMIA 2006 Annual Symposium Proceedings, 1. Januar 2006, Seite 544-548, beschreiben eine Sichtungsvorrichtung zur Sichtung von Schadensereignissen gemäß des Oberbegriffs des Anspruchs 1.

Leslie A. Lenert et al., AMIA 2005 Annual Symposium Proceedings, 1. Januar 2005, Seite 440-444 offenbaren eine Sichtungsvorrichtung zur Sichtung von Schadensereignissen, bei welcher die Kommunikation zwischen Datenübertragungseinrichtung und Empfangseinrichtung in Echtzeit erfolgt.

Aus US 2011/130636 A1 ist die Bestimmung der Position von Sichtungseinrichtungen zur Sichtung von Schadensereignissen durch Triangulation bekannt.

Problematisch an den aus dem Stand der Technik bekannten Lösungen ist zum einen, dass es den den Erstsichtern nachfolgenden Einsatzkräften nicht ohne elektronische Hilfsmittel möglich ist, den Zustand der Personen zu erfassen. Vielmehr muss immer die Vorrichtung elektronisch ausgelesen werden.

Des Weiteren ist es problematisch, dass WLAN oder Mobilfunkverbindungen oftmals im Falle eines Massenanfalls von Verletzen nicht zur Verfügung stehen oder überlastet sind. Auch weisen WLAN-Verbindungen meist eine zu geringe Reichweite auf, um praktisch einsetzbar zu sein.

Auch besteht die Gefahr der Beschädigung der Vorrichtungen, deren technischen Ausfalls aufgrund von Störungen, oder einfach einem mangelnden Ladezustand der Vorrichtungen, so dass ein Auslesen durch die nachfolgenden Einsatzkräfte nicht erfolgen kann.

Die Aufgabe der vorliegenden Erfindung liegt nunmehr darin, die Nachteile des Stands der Technik zu überwinden und insbesondere eine Vorrichtung zu liefern, die eine schnelle und präzise Übersicht über die Anzahl der betroffenen Personen im Falle eines Massenanfalls von Verletzten ermöglicht, ein einfaches Erkennen der Einschätzung des Zustands der Erstsichter durch die nachfolgenden Einsatzkräfte ohne technische Hilfsmittel erlaubt, und die gleichzeitig mindestens die gleiche Zuverlässigkeit wie die bislang verwendeten Verletztenanhängerkarten sicherstellt.

Diese Aufgabe wird gelöst durch eine Sichtungsvorrichtung zur Sichtung von Schadensereignissen umfassend ein Einstellelement zum Einstellen von mindestens zwei unterschiedlichen Zuständen einer Person und eine Datenübertragungseinrichtung, wobei jeder Zustand eine von den anderen Zuständen unterscheidbare grafische Kennzeichnung aufweist, und der eingestellte Zustand mittels der Datenübertragungseinrichtung drahtlos an eine Empfangseinrichtung übermittelbar ist bzw. übermittelt wird, und wobei die Sichtungsvorrichtung eine feste individuelle Kennung umfasst, wobei die Kennung von der Datenübertragungseinrichtung an die Empfangseinrichtung sendbar ist bzw. gesendet wird, und wobei die Kennung zusätzlich mittels eines Daten-Tags auslesbar ist bzw. ausgelesen wird, dadurch gekennzeichnet, dass das Einstellelement in Form eines Drehschalters und eines zusätzlichen Schiebeschalters ausgebildet ist, wobei der Drehschalter für jeden Zustand eine von den anderen Zuständen unterscheidbare grafische Kennzeichnung aufweist und durch den Schiebeschalter eine, insbesondere zweistufige, Priorisierung des mit dem Drehschalter eingestellten Zustands einstellbar ist.

Der Erfindung liegt dabei die überraschende Erkenntnis zugrunde, dass die Nachteile des Stands der Technik dadurch überwunden werden können, dass durch eine optische Anzeige des Zustands einer Person für die den Erstsicher folgenden Einsatzkräften entsprechend den derzeit verwendeten Verletztenanhängerkarten die bestehenden Vorteile aufrechterhalten werden können, während durch eine zusätzlich Übertragung des Zustands mittels einer Datenübertragungseinrichtung an eine Empfangseinrichtung die Informationen gleichzeitig der Einsatzleitstelle bereitgestellt werden können.

Es wird somit ermöglicht, dass der Zustand jeder begutachteten Person ohne zusätzliche Hilfsmitteln von den den Ersichtern nachfolgenden Einsatzkräften direkt erfasst werden kann, und die Anzahl der Personen für eine Planung der Anforderung weiterer Einsatzkräfte, Rettungswagen, Krankenhausbetten, etc. ohne zusätzlichen manuellen Übermittlungsaufwand bereitgestellt wird.

Des Weiteren hat es sich erfindungsgemäß als vorteilhaft erwiesen, wenn jeder Sichtungsvorrichtung eine individuelle Kennung zugeordnet wird. Während aus Sicherheitsgründen es insbesondere vorgesehen sein kann, dass die Kennung in Form einer Zahl, oder in Form von Buchstaben, oder einer Kombination von Zahlen und Buchstaben für den Menschen lesbar auf der jeweiligen Sichtungsvorrichtung angebracht ist, ist es besonders vorteilhaft, wenn ein automatisches Auslesen der individuellen Kennung ermöglicht wird.

Somit können wichtige Informationen einer Person beispielsweise von einem Notarzt zur Dokumentation durch Auslesen des Daten-Tags zugeordnet werden, ohne dass die Kennung manuell eingegeben werden muss. Das spart wertvolle Zeit. Auch kann ein Auslesen des Daten-Tags erfolgen, um ergänzende Informationen über eine Person in einer Datenbank zu hinterlegen.

Des Weiteren kann das Auslesen eines Daten-Tags und somit der aktuelle Behandlungs- bzw. Transportzustand einer Personen automatisch von der Auslesevorrichtung an die Einsatzleitstelle zur Aktualisierung übermittelt werden, so dass nicht nur der Zustand und der Ort einer Person bekannt sind und visualisiert werden können, sondern für die Einsatzplanung auch ersichtlich ist, welche Personen bereits weitergehend versorgt werden. Dies dient unter anderem der Ressourcenallokation, beispielsweise für die Anforderung weiterer Rettungswagen oder von Krankenhausbetten. Durch ein Auslesen des Datentags wird zudem die Dokumentation des Einsatzes optimiert, da jeder Schritt direkt zentral erfassbar ist. Ein weiterer Vorteil des erfindungsgemäßen Daten-Tags liegt auch darin, dass diese unabhängig von der vollen Funktionalität des Sichtungsgeräts als solches ausgelesen werden können. Auch wenn die Sichtungsgeräte selbst stabil und zuverlässig konstruiert sind, so können Beschädigungen an diesen nicht vollständig ausgeschlossen werden.

Während der Zustand des Patienten auch unabhängig von dem "Funktionieren" der Sichtungsvorrichtung anhand der farblichen Markierungen erfasst werden kann, ermöglicht der Daten-Tag, dass eine vollständige Zuordnung der Patientendaten aus dem System erfolgen kann, auch wenn die Sichtungsvorrichtung selbst nicht voll funktionsfähig oder sogar komplett ausgefallen ist, da diese mittels des Daten-Tags verknüpft sind.

Das erfindungsgemäße Sichtungsgerät ermöglicht somit ein vollständig redundantes Auslesen der Daten. Wie die konventionellen Sichtungskarten kann der Zustand anhand einer farblichen Markierung abgelesen werden. Zudem werden Daten über den Zustand einer Person an eine Zentrale übermittelt. Soll eine schnelle Zuordnung zu der Person an einer Sammelstelle etc. erfolgen, kann einfach der Daten-Tag ausgelesen werden. Dies funktioniert auch, wenn das Sichtungsgerät selbst ansonsten stark beschädigt ist.

Des Weiteren kann es insbesondere vorgesehen sein, dass die grafische Kennzeichnung in Form einer fest aufgebrachten farblichen Markierung bereitgestellt ist, wobei insbesondere das Einstellelement in Form eines Dreh- und/oder Schiebeschalters ausgebildet ist, und jeder Zustand des Dreh- oder Schiebschalters eine fest aufgebrachte farbliche Markierung anzeigt.

Es hat sich erfindungsgemäß als besonders vorteilhaft erwiesen, dass als Einstellelement ein Dreh- und/oder Schiebeschalter zum Einsatz kommt. Dieser ist durch die Erstsichter einfach bedienbar und die Gefahr einer falschen Einstellung des Zustands ist minimiert. Die Ersthelfer sind bereits mit dem System der Verletztenanhängerkarten vertraut und die Farbcodierung ist geläufig. Alternativ kann auch ein Folientaster zum Einsatz kommen, der jedoch im Vergleich zu den Dreh- und/oder Schiebeschaltern nicht ohne eine Energieversorgung betrieben werden kann.

Durch eine fest aufgebrachte farbliche Markierung je nach Stellung des Einstellelements wird zudem ermöglicht, dass selbst im Falle einer technischen Fehlfunktion der erfindungsgemäßen Sicherungsvorrichtung sichergestellt ist, dass mindestens die gleiche Funktionalität der bisher verwendeten Verletztenanhängerkarten aufrechthalten wird.

Durch eine erfindungsgemäße feste farbliche Markierung der jeweiligen Zustände werden auch von den den Erstsichtern nachfolgenden Einsatzkräften keine zusätzlichen technischen Hilfsmittel benötigt, um den Zustand einer Person zu erfassen.

Des Weiteren kann es vorteilhaft sein, dass die grafische Kennzeichnung in Form einer farblichen Markierung bereitgestellt ist, wobei der mittels des Einstellelements eingestellte Zustand mittels eines Anzeigeelements, insbesondere in Form von farblichen LEDs, angezeigt ist.

Zusätzlich oder alternativ zu der festen farblichen Markierung am Einstellelement kann die erfindungsgemäße Sichtungsvorrichtung eine Anzeige des eingestellten Zustands der Person mittels eines Anzeigeelements umfassen. Dies hat insbesondere den Vorteil, beispielsweise bei Verwendung der als besonders bevorzugt angesehen LEDs als Anzeigeelement, dass auch bei schlechten Licht- oder Wetterverhältnissen der Zustand einer Person einfach erkennbar ist. Zudem kann die Anzeigeeinrichtung der Lokalisierung von Personen dienen, da diese durch das Leuchten des Anzeigeelement schneller auffindbar sind. OLEDs haben dabei insbesondere den Vorteil, dass diese auch bei einer, ggf. zwischenzeitlichen, Unterbrechung der Energieversorgung einsatzbereit sind.

Die erfindungsgemäße Kombination von Dreh- und Schiebeschalter hat den Vorteil, dass eine weiter untergliederte Priorisierung vorgenommen werden kann, indem beispielsweise die Kategorie T1/IMMEDIATE gemäß STaRT mittels des Drehschalters eingestellt wird, und besonders kritische Patienten über den Schiebschalter hervorgehoben werden. Der Schiebschalter umfasst dabei bevorzugt zwei Stellungen, eine Ausgangsposition und eine Stellung für erhöhte Priorität.

Des Weiteren kann es sich gemäß einer Ausführungsform der vorliegenden Erfindung als vorteilhaft erweisen, dass eine Positionserfassungsvorrichtung umfasst ist, die die Position der Sichtungsvorrichtung erfasst, insbesondere in Form eines GPS-Empfängers, wobei die Daten der Positionserfassungsvorrichtung mittels der Datenübertragungseinrichtung drahtlos an die Empfangseinrichtung übermittelbar sind bzw. übermittelt werden.

Durch eine erfindungsgemäße Positionserfassungsvorrichtung kann ermöglicht werden, dass der Ort einer begutachteten Person automatisch an die Empfangseinheit übermittelt und somit der Einsatzleitstelle zur Kenntnis gebracht wird. Dies hat den Vorteil, dass automatisiert eine Karte bzw. ein Lageplan erstellt werden kann, die die Anzahl, den Zustand sowie die Position der erfassten Personen in Echtzeit abbildet.

Des Weiteren kann es vorteilhaft sein, dass die drahtlose Kommunikation der Datenübertragungseinrichtung mit der Empfangseinrichtung über ein lokales Funknetz, eine Long range, low power wireless platform, ein Long Range Wide Area Network, SigFOX, WLAN, GMS, LTE und/oder UMTS erfolgt.

Die Datenübertragungseinrichtung kann erfindungsgemäß mit der Empfangseinrichtung über verschiedene Datenübertragungprotokolle kommunizieren. Neben den weit verbreiteten Möglichkeiten einer Datenübertragung über WLAN oder Mobilfunknetze hat es sich jedoch als besonders vorteilhaft erwiesen, wenn eine Datenübertragung über ein lokales Funknetz, insbesondere über eine Long range, low power wireless platform und /oder ein Long Range Wide Area Network erfolgt.

Während im Falle eines Massenanfalls von Verletzten die Mobilfunknetze ausfallen oder aufgrund von Überlastung zusammenbrechen können und WLAN gerade bei unebenem Gelände oder innerhalb von Gebäuden nur eine sehr begrenzte Reichweite aufweist, ermöglicht eine Long range, low power wireless platform und/oder ein Long Range Wide Area Network hohe Reichweiten von bis zu mehr als 10 km für die Uplink-Kommunikation. Die Netzarchitektur einer Long range, low power wireless platform bzw. eines Long Range Wide Area Network ist dabei sternförmig, und die Endgeräte kommunizieren mit Gateways als Empfangseinrichtungen, welche die Datenpakete an einen Netzwerkserver weiterleiten.

Eine Verbindung der Gateways der Long range, low power wireless platform bzw. des Long Range Wide Area Network mit den Netzwerkservern kann dabei erfindungsgemäß bevorzugt mittels WLAN erfolgen, um einen schnellen Aufbau der Long range, low power wireless platform bzw. des Long Range Wide Area Network zu ermöglichen. Ein LoRaWan nutzt dabei regional unterschiedliche Frequenzbereiche des ISM-Bands, die üblicherweise im Bereich zwischen 433 MHz und 915 MHz liegen, und somit deutlich unter der Frequenz eines WLANs.

Eine Long range, low power wireless platform bzw. ein Long Range Wide Area Network kann dabei im Falle eines Massenanfalls von Verletzen schnell aufgebaut werden und je nach Anzahl der Gateways an unterschiedliche räumliche Begebenheiten angepasst werden, so dass eine optimale Abdeckung des Einsatzgebiets möglich ist.

Des Weiteren kann es bevorzugt sein, dass der Daten-Tag in Form eines QR-Codes, Barcodes, Strichcodes, NFC-Tags und/oder Bluetooth ausgebildet ist.

Gemäß einer Ausführungsform der Erfindung kann es zudem vorteilhaft sein, dass eine Aktivitätserfassungeinrichtung umfasst ist, die eine Bewegung der Vorrichtung erfasst, insbesondere eine Beschleunigung, und/oder eine Lage der Vorrichtung, und/oder eine Drehrate der Vorrichtung, wobei die Aktivitätserfassungsvorrichtung insbesondere einen Beschleunigungssensor und/oder einen Lagesensor und/oder einen Drehratensensor und/oder eine integrierte Variante der genannten Sensoren umfasst.

Durch eine solche Aktivitätserfassungseinrichtung wird nicht nur der Ort der Personen erkannt, sondern auch der jeweilige Bewegungszustand. Dies hat den Vorteil, dass zentral ersichtlich ist, welche Personen sich z.B. bereits eigenständig zu einer Sammelstelle bewegen, welche Personen von Rettungskräften transportiert werden, etc.

Gemäß einer vorteilhaften Weiterentwicklung der vorliegenden Erfindung kann es zudem vorgesehen sein, dass eine Vitalparametererfassungseinrichtung umfasst ist, die ausgelegt und eingerichtet ist, um Vitalparameter einer Person zu erfassen, insbesondere die Herzfrequenz, die pulsoxymetrisch gemessene Sauerstoffsättigung (SpO₂), den Blutdruck, die Körpertemperatur und/oder die Atemfrequenz.

Die automatische Erfassung der Vitalparameter einer Person kann der Überprüfung der Einschätzung der Erstsicher dienen und insbesondere auch eine Verschlechterung des Zustands einer Person für die Einsatzleitstelle anzeigen. Sofern die Vitalwerte unter bestimmte Schwellwerte gemäß der eingesetzten Klassifikation (bspw. STaRT) fallen, kann zudem eine Warnung erfolgen und/oder eine automatisierte Reklassifizierung der Person.

Dabei kann es insbesondere vorteilhaft sein, dass der eingestellte Zustand einen Zustand einer infolge eines Schadensereignisses verletzten Person repräsentiert, wobei der Zustand eine Kenngröße der Prioritätsstufe zur Versorgung der verletzten Person darstellt, insbesondere gemäß der Triagekategorie T1 bis T4.

Gemäß einer Ausführungsform der vorliegenden Erfindung kann vorgesehen sein, dass die Vorrichtung ein Befestigungsmittel zur Befestigung an einer Person umfasst.

Es kann sich erfindungsgemäß als vorteilhaft erweisen, wenn eine Befestigung der Vorrichtung mittels eines Befestigungsmittels an der Person erfolgt. Dies kann mittels einer Schlaufe, die um den Hals einer Person gelegt wird, realisiert werden, jedoch auch mit anderen gängigen Befestigungsmitteln wie Armbändern, die fest am Körper anliegen, um eine Erfassung der Vitalparameter zur ermöglichen.

Auch kann bevorzugt sein, dass die Vorrichtung wasserdicht ausgebildet ist, wobei insbesondere das Einstellelement in Form eines magnetischen Schiebe- und/oder Drehschalters ausgebildet ist.

Der Zuverlässigkeit der erfindungsgemäßen Sichtungsvorrichtung wird besondere Bedeutung beigemessen. Es hat sich gezeigt, dass besonders vorteilhafterweise die Sichtungsvorrichtung selbst wasserdicht ausgebildet ist, um beispielsweise auch bei Unwetter und starkem Regenfall zuverlässig zu funktionieren. Um dies zu ermöglichen kann es vorteilhaft sein, dass das Einstellelement die eingestellten Werte mittels eines magnetbasierten Dreh- und/oder Schiebeschalters übermittelt, da das Bedienteil des Dreh- und/oder Schiebschalters außerhalb eines gekapselten Gehäuses der Vorrichtung angeordnet ist und bedient wird.

Des Weiteren kann es bevorzugt sein, dass die Datenübertragungseinrichtung den eingestellten Zustand, die Position der Sichtungsvorrichtung, eine individuelle Kennung der Vorrichtung, die Beschleunigung der Vorrichtung, die Lage der Sichtungsvorrichtung, die Vitalparameter der Person und/oder weitere Daten zum wiederholten Senden an die Empfangseinrichtung in regelmäßigen Zeitabständen überträgt.

Erfindungsgemäß hat es sich als bevorzugt erwiesen, dass die Sichtungsvorrichtung selbst periodisch relevante Daten mittels der Datenübertragungseinrichtung an die Empfangseinrichtung überträgt, ohne dass eine Datenabfrage an die Vorrichtung gerichtet werden muss. Die automatisch ausgesendeten Daten der Sichtungsvorrichtung können dabei von einer Antenne beispielswiese des LaRoWans direkt erfasst und weitergeleitet werden.

Auch liefert die Erfindung eine Anordnung umfassend mindestens zwei erfindungsgemäße Sichtungsvorrichtungen, mindestens eine Empfangseinrichtung und mindestens einen Server, wobei der Server mittels der von der mindestens einen Empfangseinrichtung empfangenen Daten die Anzahl und den Zustand von Personen erfasst und auf einer Anzeigeeinrichtung anzeigt.

Durch eine erfindungsgemäße Anordnung wird ermöglicht, dass die Daten von mindestens zwei, insbesondere einer Vielzahl von erfindungsgemäßen Sichtungsvorrichtung aggregiert und zentral ausgewertet werden.

Dabei kann bevorzugt sein, dass die Empfangseinrichtung einer erfindungsgemäßen Sichtungsvorrichtung ein lokales Funknetz, insbesondere ein Long Range Wide Area Network, ausbilden, wobei die Vorrichtungen mit Gateways kommunizieren und die Gateways die Datenpakete an den mindestens einen Server senden.

Ein lokales Funknetz kann durch die Einsatzkräfte schnell aufgebaut werden und ermöglicht eine sichere und zuverlässige Kommunikation unabhängig von einer bestehenden Infrastruktur.

Schließlich kann vorgesehen sein, dass die Position der Sichtungsvorrichtungen mittels der Gateways der Empfangseinrichtung trianguliert wird.

Erfindungsgemäß kann es bevorzugt sein, dass die Position einer erfindungsgemäßen Sichtungsvorrichtung mittels eines von der Sichtungsvorrichtung umfassten GPS-Empfängers ermittelt wird. In bestimmen Situationen, beispielsweise wenn kein zuverlässiger GPS-Empfang möglich ist, insbesondere in unebenem Geländer oder innerhalb von Gebäuden, kann erfindungsgemäß auch alternativ oder zusätzlich eine Ortung mittels bekannten Triangulationsverfahren vorgenommen werden, wobei auf die empfangenen Signale der Gateways des lokalen Funknetzwerks zurückgegriffen wird.

Alternativ kann erfindungsgemäß auf bekannte Lokalisationsverfahren insbesondere für die (Indoor-) Lokalisation zurückgegriffen werden, wie beispielsweise auf Beacons, WLAN-Navigation und dergleichen.

Auch liefert die Erfindung eine Verwendung einer erfindungsgemäßen Sichtungsvorrichtung oder einer erfindungsgemäßen Anordnung zur Ortung und Sichtung von Schadensereignissen.

Weitere Merkmale und Vorteile der Erfindung ergeben sich aus der nachfolgenden Beschreibung, in der ein Ausführungsbeispiel der Erfindung anhand einer schematischen Zeichnung beispielhaft erläutert wird, ohne dadurch die Erfindung zu beschränken.

Dabei zeigt:

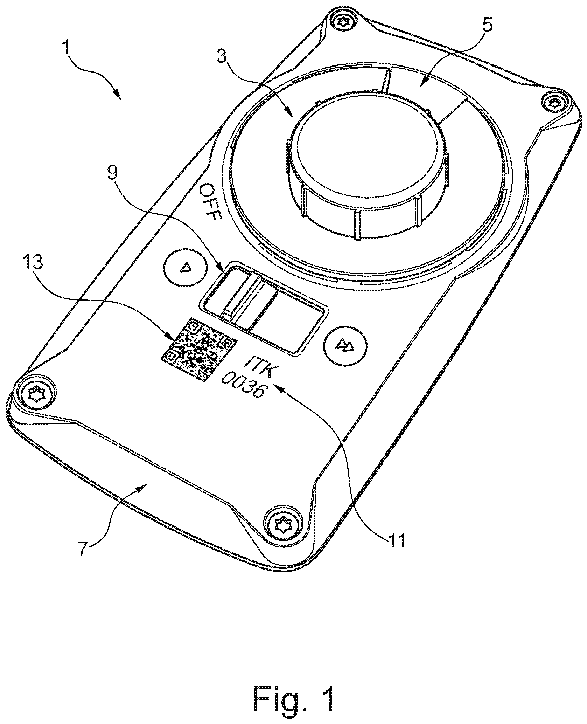

Figur 1: eine schematische Ansicht einer erfindungsgemäßen Vorrichtung.

In Figur 1 ist eine schematische Ansicht einer erfindungsgemäßen Sichtungsvorrichtung 1 gezeigt. Diese umfasst eine Drehschalter 3, mittels dessen der Zustand einer Person definiert werden kann. Der jeweilige Zustand der Person ist dabei für einen Benutzer, insbesondere den Erstsichter und die nachfolgenden Einsatzkräfte, einfach durch eine farbliche Markierung ersichtlich.

Parallel wird der Zustand mittels einer nicht gezeigten Datenübertragungseinrichtung an eine Empfangseinrichtung drahtlos übertragen.

Um insbesondere bei schlechten Sichtverhältnissen, beispielsweise in der Nacht oder bei Regen, den Zustand möglichst schnell den Einsatzkräften zu Kenntnis zu bringen, ist zudem eine Anzeigeeinrichtung 7 umfasst, die gemäß dem vorliegenden Ausführungsbeispiel mittels farbiger LEDs bereitgestellt werden kann.

Des Weiteren umfasst die Vorrichtung 1 gemäß dem vorliegenden Ausführungsbeispiel einen Schiebeschalter 9, der der weiteren Priorisierung der Personen dienen kann. Personen in einem besonders kritischen Zustand können dabei durch ein Verschieben des Schiebeschalters 9 von der dargestellten Position des Schiebschalters 9 in die gegenüberliegende Position markiert werden.

Des Weiteren umfasst die Vorrichtung 1 eine aufgedruckte individuelle Markierung 11, hier eine Nummer, um die Sichtungsvorrichtung 1 eindeutig zu identifizieren.

Für ein schnelles Zuordnen der Vorrichtung kann zudem eine eindeutige Identifikation der Vorrichtung 1 durch einen Daten-Tag 13, hier in Form eines QR-Codes, erfolgen.

## Patentansprüche

1. Sichtungsvorrichtung zur Sichtung von Schadensereignissen umfassend ein Einstellelement zum Einstellen von mindestens zwei unterschiedlichen Zuständen einer Person und eine Datenübertragungseinrichtung, wobei jeder Zustand eine von den anderen Zuständen unterscheidbare grafische Kennzeichnung aufweist, und der eingestellte Zustand mittels der Datenübertragungseinrichtung drahtlos an eine Empfangseinrichtung übermittelbar ist bzw. übermittelt wird, und wobei die Sichtungsvorrichtung eine feste individuelle Kennung umfasst, wobei die Kennung von der Datenübertragungseinrichtung an die Empfangseinrichtung sendbar ist bzw. gesendet wird, und wobei die Kennung zusätzlich mittels eines Daten-Tags auslesbar ist bzw. ausgelesen wird, **dadurch gekennzeichnet, dass** das Einstellelement in Form eines Drehschalters und eines zusätzlichen Schiebeschalters ausgebildet ist, wobei der Drehschalter für jeden Zustand eine von den anderen Zuständen unterscheidbare grafische Kennzeichnung aufweist und durch den Schiebeschalter eine, insbesondere zweistufige, Priorisierung des mit dem Drehschalter eingestellten Zustands einstellbar ist.

2. Sichtungsvorrichtung nach Anspruch 1, **dadurch gekennzeichnet, dass** die grafische Kennzeichnung in Form einer fest aufgebrachten farblichen Markierung bereitgestellt ist, wobei insbesondere das Einstellelement in Form eines Dreh- und/oder Schiebeschalters ausgebildet ist, und jeder Zustand des Dreh- oder Schiebschalters eine fest aufgebrachte farbliche Markierung anzeigt, so dass insbesondere die Sichtungsvorrichtung selbst dann noch das Ziel der Bewältigung eines Massenanfalls von Verletzten erfüllt, wenn die Sichtungsvorrichtung selbst nicht voll funktionsfähig oder sogar komplett ausgefallen ist.

3. Sichtungsvorrichtung nach Anspruch 1 oder Anspruch 2, **dadurch**
**gekennzeichnet, dass**
die grafische Kennzeichnung in Form einer farblichen Markierung bereitgestellt ist, wobei der mittels des Einstellelements eingestellte Zustand mittels eines Anzeigeelements, insbesondere in Form von farblichen LEDs und/oder OLEDs, angezeigt ist.

4. Sichtungsvorrichtung nach einem der vorangehenden Ansprüche, **dadurch**
**gekennzeichnet, dass**
die Kommunikation zwischen der Datenübertragungseinrichtung und der Empfangseinrichtung in Echtzeit erfolgt, so dass insbesondere die Schadenslage in Echtzeit erfassbar ist.

5. Sichtungsvorrichtung nach einem der vorangehenden Ansprüche, **dadurch**
**gekennzeichnet, dass**
eine Positionserfassungsvorrichtung umfasst ist, die die Position der Vorrichtung erfasst, insbesondere in Form eine GPS-Empfängers, wobei die Daten der Positionserfassungsvorrichtung mittels der Datenübertragungseinrichtung drahtlos an die Empfangseinrichtung übermittelbar sind bzw. übermittelt werden.

6. Sichtungsvorrichtung nach einem der vorangehenden Ansprüche, **dadurch**
**gekennzeichnet, dass**
die drahtlose Kommunikation der Datenübertragungseinrichtung mit der Empfangseinrichtung über ein lokales Funknetz, eine Lang range, low power wireless platform, ein Lang Range Wide Area Network, SigFOX, WLAN, GSM, LTE und/oder UMTS erfolgt.

7. Sichtungsvorrichtung nach einem der vorangehenden Ansprüche, **dadurch**
**gekennzeichnet, dass**
der Datentag in Form eines QR-Codes, Barcodes, Strichcodes, NFC-Tags und/oder eines Blue-Tooth-Tags ausgebildet ist.

8. Sichtungsvorrichtung nach einem der vorangehenden Ansprüche, **dadurch**
**gekennzeichnet, dass**
eine Aktivitätserfassungseinrichtung umfasst ist, die eine Bewegung der Vorrichtung erfasst, insbesondere eine Beschleunigung, und/oder eine Lage der Vorrichtung, und oder eine Drehrate der Vorrichtung, wobei die Aktivitätserfassungsvorrichtung insbesondere einen Beschleunigungssensor und/oder einen Lagesensor und/oder einen Drehratensensor und/oder eine integrierte Variante der genannten Sensoren umfasst.

9. Sichtungsvorrichtung nach einem der vorangehenden Ansprüche, **dadurch**
**gekennzeichnet, dass**
eine Vitalparametererfassungseinrichtung umfasst ist, die ausgelegt und eingerichtet ist, um Vitalparameter einer Person zu erfassen, insbesondere die Herzfrequenz, die pulsoxymetrisch gemessene Sauerstoffsättigung (SpO2), den Blutdruck, die Körpertemperatur und/oder die Atemfrequenz.

10. Sichtungsvorrichtung nach einem der vorangehenden Ansprüche, **dadurch**
**gekennzeichnet, dass**
der eingestellte Zustand einen Zustand einer infolge eines Schadensereignisses verletzten Person repräsentiert, wobei der Zustand eine Kenngröße der Prioritätsstufe zur Versorgung der verletzten Person darstellt, insbesondere gemäß international anerkannter Sichtungskategorien und/oder Triagekategorien.

11. Sichtungsvorrichtung nach einem der vorangehenden Ansprüche, **dadurch**
**gekennzeichnet, dass**
die Sichtungsvorrichtung ein Befestigungsmittel zur Befestigung an einer Person umfasst.

12. Sichtungsvorrichtung nach einem der vorangehenden Ansprüche, **dadurch**
**gekennzeichnet, dass**
die Sichtungsvorrichtung wasserdicht ausgebildet ist, wobei insbesondere das Einstellelement in Form eines magnetischen Schiebe- und/oder Drehschalters ausgebildet ist.

13. Sichtungsvorrichtung nach einem der vorangehenden Ansprüche, **dadurch**
**gekennzeichnet, dass**
die Datenübertragungseinrichtung den eingestellten Zustand, die Position der Sichtungsvorrichtung, eine individuelle Kennung der Vorrichtung, die Beschleunigung der Sichtungsvorrichtung, die Lage der Sichtungsvorrichtung, die Vitalparameter der Person und/oder weitere Daten zum wiederholten Senden an die Empfangseinrichtung in regelmäßigen Zeitabständen überträgt.

14. Anordnung umfassend mindestens zwei Sichtungsvorrichtungen nach einem der vorangehenden Ansprüche, mindestens eine Empfangseinrichtung und mindestens einen Server, wobei der Server mittels der von der mindestens einen Empfangseinrichtung empfangenen Daten die Anzahl und den Zustand von Personen erfasst und auf einer Anzeigeeinrichtung anzeigt.

15. Anordnung nach Anspruch 14, **dadurch gekennzeichnet, dass**
die Empfangseinrichtung ein lokales Funknetz, insbesondere ein Lang range, low power wireless platform, ein Lang Range Wide Area Network, ein SigFOX-Netzwerk, und/oder ein Lang Range Wide Area Network, ausbilden, wobei die Sichtungsvorrichtungen mit Gateways kommunizieren und die Gateways die Datenpakete an den mindestens einen Server senden.

16. Anordnung nach Anspruch 14 oder Anspruch 15, **dadurch gekennzeichnet,**
**dass**
die Position der Sichtungsvorrichtungen mittels der Gateways der Empfangseinrichtung trianguliert wird.

17. Verwendung einer Sichtungsvorrichtung nach einem der Ansprüche 1 bis 13 oder einer Anordnung gemäß einem der Ansprüche 14 bis 16 zur Ortung und Sichtung von Schadensereignissen.

## Claims

1. Screening apparatus for screening injury incidents, comprising a setting element for setting at least two different states of a person and a data transmission device, wherein each state comprises a graphical identification that can be distinguished from the other states, and the set state can be transmitted or is transmitted wirelessly to a receiving device by means of the data transmission device, and wherein the screening apparatus comprises a fixed individual identifier, wherein the identifier can be transmitted or is transmitted from the data transmission device to the receiving device, and wherein the identifier additionally can be read out or is read out by means of a data tag, **characterized in that** the setting element is in the form of a rotary switch and an additional sliding switch, wherein the rotary switch has for each state a graphical identification that can be distinguished from the other states and the sliding switch can be used to set an, in particular two-stage, prioritization of the state that is set using the rotary switch.

2. Screening apparatus according to Claim 1, **characterized in that** the graphical identification is provided in the form of a fixedly applied colour marker, wherein the setting element is, in particular, in the form of a rotary and/or sliding switch, and each state of the rotary or sliding switch displays a fixedly applied colour marker such that, in particular, the screening apparatus itself then still achieves the aim of managing a mass casualty incident if the screening apparatus itself is not fully functional or even has completely failed.

3. Screening apparatus according to Claim 1 or Claim 2, **characterized in that** the graphical identification is provided in the form of a colour marker, wherein the state that is set by means of the setting element is displayed by means of a display element, in particular in the form of colour LEDs and/or OLEDs.

4. Screening apparatus according to any one of the preceding claims, **characterized in that** the communication between the data transmission device and the receiving device takes place in real time such that, in particular, the injury situation can be acquired in real time.

5. Screening apparatus according to any one of the preceding claims, **characterized in that** a position detection apparatus is included, which detects the position of the apparatus, in particular in the form of a GPS receiver, wherein the data from the position detection apparatus can be transmitted or are transmitted wirelessly to the receiving device by means of the data transmission device.

6. Screening apparatus according to any one of the preceding claims, **characterized in that** the wireless communication between the data transmission device and the receiving device takes place via a local radio network, a long range, low power wireless platform, a long range wide area network, SigFOX, WLAN, GSM, LTE and/or UMTS.

7. Screening apparatus according to any one of the preceding claims, **characterized in that** the data tag is in the form of a QR code, barcode, NFC tag and/or a Bluetooth tag.

8. Screening apparatus according to any one of the preceding claims, **characterized in that** an activity detection device is included, which detects a movement of the apparatus, in particular an acceleration, and/or a location of the apparatus, and/or a rate of rotation of the apparatus, wherein the activity detection device comprises, in particular, an acceleration sensor and/or a location sensor and/or a rate of rotation sensor and/or an integrated variant of said sensors.

9. Screening apparatus according to any one of the preceding claims, **characterized in that** a vital parameter detection device is included, which is designed and set up to detect vital parameters of a person, in particular heart rate, oxygen saturation measured by a pulse oximeter (SpO2), blood pressure, body temperature and/or respiratory rate.

10. Screening apparatus according to any one of the preceding claims, **characterized in that** the set state represents a state of a person injured as a result of an injury incident, wherein the state constitutes a characteristic variable of the priority level for treating the injured person, in particular in accordance with internationally recognized screening categories and/or triage categories.

11. Screening apparatus according to any one of the preceding claims, **characterized in that** the screening apparatus comprises a fastening means for fastening to a person.

12. Screening apparatus according to any one of the preceding claims, **characterized in that** the screening apparatus is designed to be watertight, wherein, in particular, the setting element is in the form of a magnetic sliding and/or rotary switch.

13. Screening apparatus according to any one of the preceding claims, **characterized in that** the data transmission device transmits the set state, the position of the screening apparatus, an individual identifier of the apparatus, the acceleration of the screening apparatus, the location of the screening apparatus, the vital parameters of the person and/or other data for repeated transmission to the receiving device at regular time intervals.

14. Arrangement comprising at least two screening apparatuses according to any one of the preceding claims, at least one receiving device and at least one server, wherein the server detects the number and the state of persons by means of the data received by the at least one receiving device and displays same on a display device.

15. Arrangement according to Claim 14, **characterized in that** the receiving device forms a local radio network, in particular a long range, low power wireless platform, a long range wide area network, a SigFOX network and/or a long range wide area network, wherein the screening apparatuses communicate with gateways and the gateways transmit the data packets to the at least one server.

16. Arrangement according to Claim 14 or Claim 15, **characterized in that** the position of the screening apparatuses is triangulated by means of the gateways of the receiving device.

17. Use of a screening apparatus according to any one of Claims 1 to 13 or an arrangement according to any one of Claims 14 to 16 for locating and screening injury incidents.

## Revendications

1. Système de triage pour le triage de sinistres, comprenant un élément de réglage destiné au réglage d'au moins deux états différents d'une personne et un dispositif de transmission de données, chaque état présentant un signe distinctif graphique différentiable des autres états, et l'état réglé pouvant être communiqué ou étant communiqué sans fil à un dispositif de réception au moyen du dispositif de transmission de données, et le système de triage comportant à identifiant individuel, l'identifiant pouvant être envoyé ou étant envoyé par le dispositif de transmission de données au dispositif de réception, et l'identifiant, en plus, pouvant être lue ou étant lu au moyen d'une étiquette de données, **caractérisé en ce que** l'élément de réglage est réalisé sous la forme d'un commutateur rotatif et d'un commutateur à glissière supplémentaire, le commutateur rotatif possédant, pour chaque état, un signe distinctif graphique différentiable des autres états et une priorisation, notamment à deux niveaux, de l'état réglé avec le commutateur rotatif pouvant être réglée par le commutateur à glissière.

2. Système de triage selon la revendication 1, **caractérisé en ce que** le signe distinctif graphique est fourni sous la forme d'un marquage en couleur appliqué à demeure, l'élément de réglage étant notamment réalisé sous la forme d'un commutateur rotatif et/ou à glissière, et chaque état du commutateur rotatif ou à glissière indiquant un marquage en couleur appliqué à demeure, de sorte que notamment le système de triage lui-même remplit encore l'objectif de la maîtrise d'une situation à multiples victimes lorsque le système de triage lui-même n'est pas pleinement fonctionnel ou même complètement tombé en panne.

3. Système de triage selon la revendication 1 ou la revendication 2, **caractérisé en ce que** le signe distinctif graphique est fourni sous la forme d'un marquage en couleur, l'état réglé au moyen de l'élément de réglage étant indiqué au moyen d'un élément indicateur, notamment sous la forme de LED et/ou d'OLED en couleur.

4. Système de triage selon l'une des revendications précédentes, **caractérisé en ce que** la communication entre le dispositif de transmission de données et le dispositif de réception s'effectue en temps réel, de sorte que la situation de dommage peut notamment être détectée en temps réel.

5. Système de triage selon l'une des revendications précédentes, **caractérisé en ce qu'**un arrangement de détection de position est compris, lequel détecte la position du système, notamment sous la forme d'un récepteur GPS, les données de l'arrangement de détection de position pouvant être communiquées ou étant communiquées sans fil au dispositif de réception au moyen du dispositif de transmission de données.

6. Système de triage selon l'une des revendications précédentes, **caractérisé en ce que** la communication sans fil du dispositif de transmission de données avec le dispositif de réception s'effectue par le biais d'un réseau radioélectrique local, une plateforme sans fil à longue portée et faible consommation, un réseau étendu à longue portée, SigFOX, WLAN, GSM, LTE et/ou UMTS.

7. Système de triage selon l'une des revendications précédentes, **caractérisé en ce que** l'étiquette de données est réalisée sous la forme d'un code QR, d'un code à barres, d'une étiquette NFC et/ou d'une étiquette Bluetooth.

8. Système de triage selon l'une des revendications précédentes, **caractérisé en ce qu'**un dispositif de détection d'activité est compris, lequel détecte un mouvement du système, notamment une accélération, et/ou une position du système et/ou une vitesse de rotation du système, le dispositif de détection d'activité comprenant notamment un capteur d'accélération et/ou un capteur de position et/ou un capteur de vitesse de rotation et/ou une variante intégrée desdits capteurs.

9. Système de triage selon l'une des revendications précédentes, **caractérisé en ce qu'**un dispositif de détection des paramètres vitaux est compris, lequel est adapté et conçu pour détecter les paramètres vitaux d'une personne, notamment la fréquence cardiaque, la saturation en oxygène mesurée par oxymétrie du pouls (Sp02), la pression artérielle, la température corporelle et/ou la fréquence respiratoire.

10. Système de triage selon l'une des revendications précédentes, **caractérisé en ce que** l'état réglé représente un état d'une personne blessée en conséquence d'un sinistre, l'état représentant une grandeur caractéristique du niveau de priorité pour l'assistance de la personne blessée, notamment conformément aux catégories de triage et/ou aux catégories de classification reconnues internationalement.

11. Système de triage selon l'une des revendications précédentes, **caractérisé en ce que** le système de triage comprend un moyen de fixation servant à la fixation à une personne.

12. Système de triage selon l'une des revendications précédentes, **caractérisé en ce que** le système de triage est de configuration étanche à l'eau, l'élément de réglage étant notamment réalisé sous la forme d'un commutateur à glissière et/ou rotatif magnétique.

13. Système de triage selon l'une des revendications précédentes, **caractérisé en ce que** le dispositif de transmission de données transmet l'état réglé, la position du système de triage, un identifiant individuel du système, l'accélération du système de triage, la posture du système de triage, les paramètres vitaux de la personne et/ou des données supplémentaires à intervalles de temps réguliers en vue d'une émission répétée au dispositif de réception.

14. Arrangement comprenant au moins deux systèmes de triage selon l'une des revendications précédentes, au moins un dispositif de réception et au moins un serveur, le serveur acquérant le nombre et l'état de personnes au moyen des données reçues par l'au moins un dispositif de réception et les affichant sur un dispositif d'affichage.

15. Arrangement selon la revendication 14, **caractérisé en ce qu'**un réseau radioélectrique local, notamment une plateforme sans fil à longue portée et faible consommation, un réseau étendu à longue portée, un réseau SigFOX et/ou un réseau étendu à longue portée forment le dispositif de réception, les systèmes de triage communiquant avec des passerelles et les passerelles envoyant les paquets de données à l'au moins un serveur.

16. Arrangement selon la revendication 14 ou la revendication 15, **caractérisé en ce que** la position des systèmes de triage est triangulée au moyen des passerelles du dispositif de réception.

17. Utilisation d'un système de triage selon l'une des revendications 1 à 13 ou d'un arrangement selon l'une des revendications 14 à 16 pour la localisation et le triage de sinistres.
